# EUROPÄISCHE PATENTSCHRIFT

(11) **EP 0 639 356 B1**
(45) Veröffentlichungstag und Bekanntmachung des Hinweises auf die Patenterteilung: **05.07.2000**
(21) Anmeldenummer: 93810583.0
(22) Anmeldetag: 18.08.1993
(51) Int. Cl.: A61F 2/30, B21D 31/00

(54) **Verfahren zur Erzeugung von äusseren Verankerungsflächen an Gelenkimplantaten**
Process for shaping external anchoring surfaces for joint implants
Procédé pour former des surfaces d'ancrage en saillie sur des implants d'articulations

(43) Veröffentlichungstag der Anmeldung: 22.02.1995
(73) Patentinhaber: Sulzer Orthopädie AG, 6340 Baar (CH)
(72) Erfinder: Oehy, Jürg, CH-8405 Winterthur (CH); Yurtsever, Mustafa, CH-8280 Kreuzlingen (CH)
(74) Vertreter: Sulzer Management AG

(56) Entgegenhaltungen:
- EP-A- 0 108 729
- EP-A- 0 169 978
- EP-A- 0 186 471
- DE-A- 2 914 513
- US-A- 4 865 603

## Beschreibung

Die Erfindung handelt von einem Verfahren zur Erzeugung von äusseren Verankerungsflächen an Gelenkimplantaten aus einem plastisch verformbaren Metall, wobei die Verankerungsflächen in einem Winkel α zwischen 0 und 90 Grad zu einer Einbring- und Befestigungsrichtung des Gelenkimplantats stehen.

Gelenkimplantate müssen an ihren Verankerungsflächen zum Knochengewebe in verschiedenen Richtungen Kräfte übernehmen. Die Verankerungsflächen sollten daher in möglichst vielen Richtungen Kräfte übertragen können, ohne dass eine Lockerung vom Implantat einsetzt. Unter diesem Aspekt wurden verschiedene Texturen für Verankerungsflächen entwickelt. So zeigt die EP 0 186 471 eine Kniegelenkprothese mit sägezahnartigen Rillen, die quer zur Einbringrichtung angebracht sind, wobei die Stabilität in der Richtung der Rillen reduziert ist. Eine weitere Alternative sieht an der Prothese widerhakenähnliche Stifte vor, welche in Einbringrichtung in den Femurstumpf eindringen. Eine generelle Anforderung an Verankerungsflächen dieser Art ist, dass sie einmal eine dem Einwachsen förderliche Struktur aufweisen sollten und dass sie andererseits wirtschaftlich herstellbar sein sollten. Die DE 29 14 513 zeigt Verankerungsflächen, die Angaben zur Erzeugung und zu den absoluten Dimensionen dieser Struktur gemacht. In den relativen geometrischen Verhältnissen werden allseitig verrundete Formen vorgeschlagen, die nur eine begrenzte Primärverankerung zulassen. Eine weitere Struktur wird in der EP 0 381 351 vorgeschlagen, bei der an einer Hüftgelenkschale an einem gegen aussen vorstehenden Wulst durch schräg zum Äquator verlaufende und in Umfangsrichtung versetzte Einstiche, die sich kreuzen, eine Art randrierte Verankerungsfläche mit abgeplatteten vorstehenden Pyramiden erzeugt wird. Auch hier ist die Primärverankerung begrenzt, d.h. sehr stark von der Vorspannung im Knochenhohlraum respektive vom Untermass des Knochenhohlraums im Bereich des Äquators abhängig.

In der EP-A-0 169 978 ist eine aufspreizbare Aussenschale für eine künstliche Hüftgelenkpfanne gezeigt. Aussenschale und Pfanne besitzen an ihrer gemeinsamen Trennfläche jeweils eine Mikroverzahnung, um die Aussenschale in aufgespreizter Form gegen Kollabieren zu sichern. Auf der Aussenschale ist eine Vielzahl von vorstehenden Stiften für die Primärverankerung der Schale angebracht. Zur Vergrösserung der Anwachsoberfläche ist die Aussenschale in Teilbereichen mit in Umfangsrichtung verlaufenden Rillen versehen.

Aufgabe der Erfindung ist es Verankerungsflächen zu schaffen, bei denen eine gute Verankerung mit einem kostengünstigen Herstellverfahren erreichbar ist. Diese Aufgabe wird durch die Merkmale der Ansprüche 1 und 2 gelöst. Sie wird z.B. dadurch gelöst, dass in einem Schritt I an einem Rohling in den Verankerungsflächen Einstiche quer zur Einbring- und Befestigungsrichtung durch Spanen, Giessen oder Pressen erzeugt werden, sodass spitzwinklige Schultern stehen bleiben, welche eine Höhe in einem Betrag zwischen 0,3 und 2 mm aufweisen, während der Abstand zweier benachbarter Schulterspitzen annähernd dem Betrag der zweifachen Höhe entspricht, und dass in einem Schritt II der Rohling mit einem Presswerkzeug, dessen Grundfläche der Hüllfläche an den Verankerungsflächen des Rohlings entspricht und aus dessen Grundfläche Rippen vorstehen, welche mit ihren Mittelebenen senkrecht zur Grundfläche und parallel zur Einbring- und Befestigungsrichtung verlaufen, durch Pressen in der Einbring- und Befestigungsrichtung soweit verformt wird, dass mit den Rippen quer zu den Schultern Gräben erzeugt werden, welche Schulterabschnitte stehen lassen, wobei die Schulterränder im Bereich der Gräben entgegen der Einbring- und Befestigungsrichtung plastisch deformiert werden, um auf diese Weise aus den Schulterabschnitten Becherformen zu erzeugen, welche entgegen der Einbring- und Befestigungsrichtung geöffnet sind.

Die Erfindung hat den Vorteil, dass durch eine für die die plastische Verformung vorbereitete Geometrie an den Verankerungsflächen und eine mit einfachen Mitteln daran anschliessende Verformung komplexe Formen herstellbar sind, die für eine Verankerung im Knochengewebe besonders geeignet sind und die mit anderen Verfahren kaum erreichbar sind. Bezogen auf die Einbring- und Befestigungsrichtung wird eine Vielzahl von Bechern an den Verankerungsflächen, welche in einem Winkel α zwischen 0 und 90 Grad zur Einbring- und Befestigungsrichtung stehen, erzeugt. Der einzelne Becher ist dabei wie der Becher eines Peltonrades entgegen der Einbring- und Befestigungsrichtung geöffnet. Er stützt sich mit der hohlen Innenseite im Knochengewebe entgegen der Einbring- und Befestigungsrichtung ab. Durch die gekrümmte Innenfläche der Becher und durch die mit der Herstellung der Becherform entstandenen Gräben zwischen den Bechern wird ein seitliches Weggleiten der Verankerungsfläche quer zur Einbring- und Befestigungsrichtung verhindert. Dabei sind die absoluten Dimensionen der Becherform so gewählt, dass sich Knochengewebe unter Vorspannung in die Bereiche zwischen den Bechern hineindeformiert, um eine gute Primärverankerung entgegen der Einbring- und Befestigungsrichtung zu erreichen. Ein Vorteil des Verfahrens besteht darin, dass eine endgültige Becherform entsteht, ohne dass für diese eine entsprechende Erzeugende zur Herstellung notwendig ist. Dadurch dass quer zur Einbring- und Befestigungsrichtung in den Verankerungsflächen Einstiche und Schultern erzeugt werden, dass die Abstände der Schultern etwa dem Zweifachen ihrer Höhe entsprechen und dass die Schultern im Querschnitt spitzwinklig sind, lassen sich diese in Querrichtung, d.h. entgegen der Einbring- und Befestigungsrichtung plastisch deformieren, um Becherformen zu erzeugen. Dabei ist es wichtig, dass die Höhe der Schultern zwischen 0,3 und 2 mm beträgt, um eine der Knochenstruktur günstig angepasste Grösse der Verankerungsgeometrie zu erhalten. Die Schultern können durch Spanen, Giessen oder Pressen am Rohling erzeugt werden. In einem weiteren Schritt wird der Rohling mit einem Presswerkzeug, das Rippen quer zu den Schultern aufweist verformt, wobei die Mittelebenen der Rippen senkrecht zur Grundfläche und parallel zur Einbring- und Befestigungsrichtung verlaufen. Bei einem Winkel von 0 < α < 90 Grad der Grundfläche zur Einbring- und Befestigungsrichtung entsteht durch die Rippen ein schleifender Schnitt der die Schultern entgegen der Einbring- und Befestigungsrichtung in Schulterabschnitte auftrennt, und letztere an ihren Rändern zu den so entstandenen Gräben entgegen der Einbring- und Befestigungsrichtung zu Bechern verformt. Die Ränder können dabei bis in die Ebene der nachfolgenden Schulter herabgebogen werden. Die Grösse der Verformung muss dabei der plastischen Verformbarkeit des Grundmaterials angepasst werden. Für Reintitan, das ein gängiger Werkstoff für Verankerungsflächen ist, sind Verformungen im Betrag der Schulterhöhe möglich. Die so in der Feinstruktur entstandenen Gräben lassen beim Einbringen in das Knochenbett Rippen stehen, die das Implantat bis zur Verankerung führen und gleichzeitig eine Verschiebung in der Richtung der Schultern verhindern.

Im Fall einer femurseitigen Verankerungsfläche an einem Kniegelenkimplantat entspricht das Presswerkzeug dem zur Implantation vorbereiteten Femurstumpf, wobei aus den den Verankerungsflächen entsprechende Flächen die mit ihren Mittelebenen zur Einbring- und Verankerungsrichtung parallelen Rippen herausragen.

Im Fall einer äusseren Hüftgelenkschale entspricht das Presswerkzeug einem beispielsweise halbkugelförmigen Knochenbett, wobei an den Medianen Rippen vorstehen, deren Anzahl am Umfang mit Annäherung an dem Äquator zunimmt. Die Einbring- und Befestigungsrichtung fällt dann mit der Polachse zusammen. Diese relativ einfache Gestaltung des Presswerkzeugs, das zum Beispiel mittels Funkenerosion herstellbar ist, ermöglicht eine günstige Massenfertigung für die relativ komplexen Becherformen in den Verankerungsflächen eines Implantats.

Im folgenden ist die Erfindung anhand von Ausführungsbeispielen beschrieben. Es zeigen:
- Fig. 1: schematisch den Schnitt durch eine äussere Hüftgelenkschale, die eine Verankerungsfläche mit becherförmigen Schulterabschnitten entgegen der Einbring- und Verankerungsrichtung aufweist;
- Fig. 2: schematisch eine Draufsicht auf die Hüftgelenkschale von Fig. 1, wobei die Schultern und die in Meridianen verlaufenden Gräben durch Striche angedeutet sind;
- Fig. 3: schematisch einen vergrösserten Schnitt durch die mit Einstichen vorbearbeitete Verankerungsfläche an einem Rohling einer äusseren Hüftgelenkschale;
- Fig. 4: schematisch den Ausschnitt eines Presswerkzeugs mit Rippen, deren Mittelebenen senkrecht zur Grundfläche und parallel zur Einbring- und Befestigungsrichtung verlaufen;
- Fig. 5: schematisch den Ausschnitt einer Verankerungsfläche mit becherförmigen Schulterabschnitten;
- Fig. 6 und 7: schematisch einen Schnitt durch die becherförmigen Verankerungsflächen in Fig. 5, wobei die Ränder der Schultern unterschiedlich weit entgegen der Einbring- und Befestigungsrichtung zur nächsten Schulter heruntergezogen sind;
- Fig. 8: eine mögliche Arbeitsfolge für die Formgebung an einer äusseren Hüftgelenkschale mit becherförmigen Verankerungsflächen;
- Fig. 9: schematisch die strukturierte Verankerungsfläche mit Becherform an einer femurseitigen Kniegelenkprothese mit dem vorbearbeiteten Femur und dem diesen entsprechenden Presswerkzeug und
- Fig. 10: den Ausschnitt eines mit Einstichen und Schultern vorbearbeiteten Rohlings gemäss Fig. 9 und die nach dem Eingriff des Presswerkzeugs verformten Schulterabschnitte.

Die Figuren zeigen Verfahrensschritte zur Erzeugung von Verankerungsflächen 3 an metallischen Gelenkimplantaten, die in einem Winkel 0 < α < 90 Grad entgegen der Einbring- und Befestigungsrichtung 40 stehen und becherförmige Schulterabschnitte 14 aufweisen, welche zur Verankerung entgegen der Einbring- und Befestigungsrichtung 40 geöffnet sind. In einem ersten Schritt werden durch Spanen, Giessen oder Fressen Einstiche 6 und Schultern 8 in den Verankerungsflächen 3 eines Rohlings 2 quer zur Einbring- und Befestigungsrichtung 40 erzeugt. Die Schultern 8 sind spitzwinklig im Querschnitt, haben eine Höhe 10 zwischen 0,3 und 2 mm und einen Abstand 31 der annähernd dem Betrag der zweifachen Höhe 10 entspricht. In einem zweiten Schritt werden mit einem Presswerkzeug 13, das zu den Verankerungsflächen 3 Gegenflächen aufweist, welche mit vorstehenden Rippen 26 versehen sind, die mit ihren Mittelebenen senkrecht zur Gegenfläche und parallel zur Einbring- und Befestigungsrichtung 40 stehen, Gräben 15 durch die Schultern 8 erzeugt. Dabei verformen sich die Ränder 17 der Schulterabschnitte 14 und es entstehen becherartige entgegen der Einbring- und Befestigungsrichtung 40 geöffnete Formen 18 zur Abstützung.

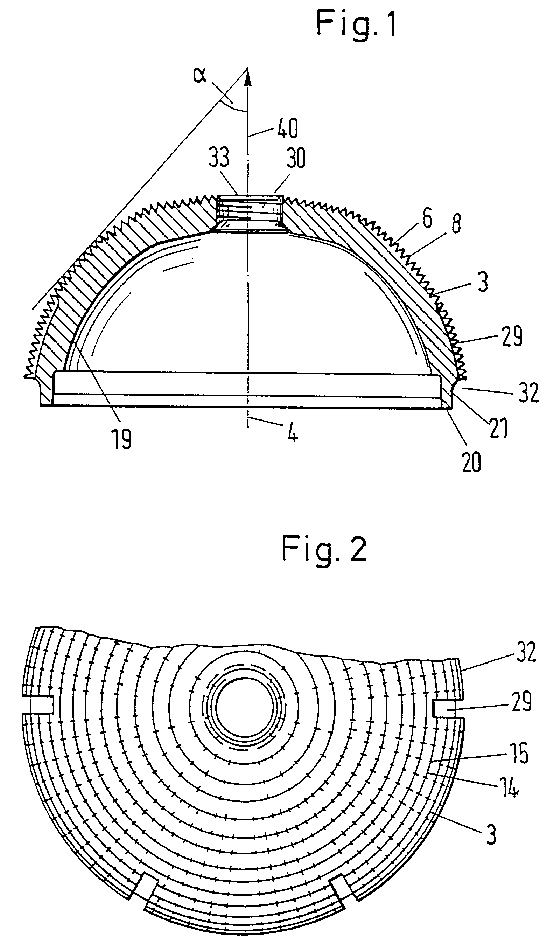

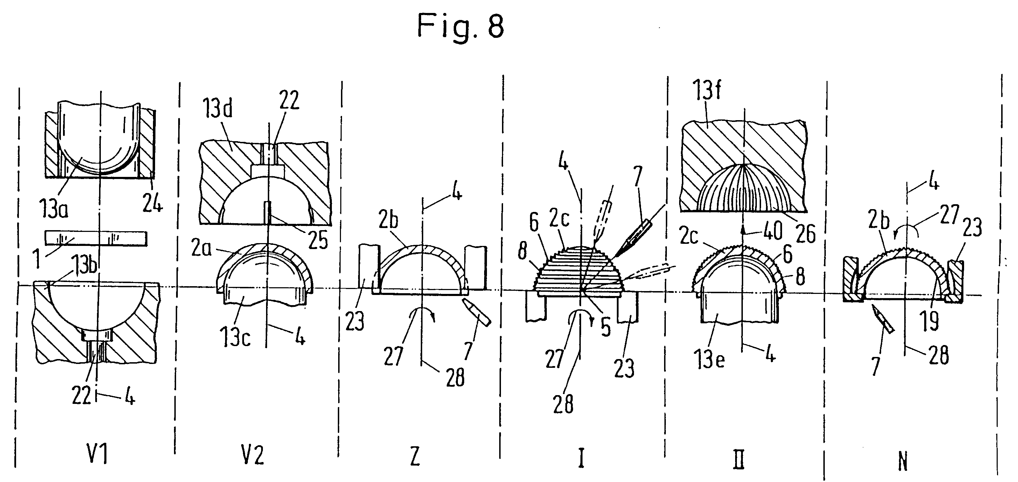

In den Figuren 1 und 2 ist eine fertig bearbeitete äussere Hüftgelenkschale gezeigt, die bezüglich ihrer Formgebung nach den in Figur 8 angedeuteten Fertigungsschritten bearbeitet wurde. Sie besitzt eine halbkugelähnliche Innenkontur 19 für die Aufnahme einer hier nicht gezeigten Innenschale, welche mit einer Schnappverbindung am Äquator 32 einrastet. In der Polachse 4 liegt am Pol 33 eine Zentrierbohrung 30 mit Innengewinde für ein Einschlagwerkzeug. Am Äquator bestehen Aufnahmeflächen 20, 21, an denen die Aussenschale zur Bearbeitung der Innenkontur 19 mit einem Spannwerkzeug aufgenommen werden kann. Ueber den Umfang sind sechs Längsrillen 29 verteilt, die der zusätzlichen Drehsicherung bei der Verankerung der Aussenschale dienen. Entsprechend Figur 8 können diese Längsschlitze mit einem Tiefziehwerkzeug 13d in einem Vorschritt V2 aufgebracht werden. Die Struktur der eigentlichen Verankerungsfläche 3 ergibt sich aus Einstichen 6 und vorstehenden Schultern 8, welche quer zur Einbring- und Befestigungsrichtung auf bestimmten Breitengraden angeordnet sind. Durch ein Tiefziehwerkzeug 13f mit in Richtung der Meridiane vorstehenden Rippen sind Gräben 15 eingepresst worden, die von den Schultern 8 nur noch Schulterabschnitte 14 stehenlassen.

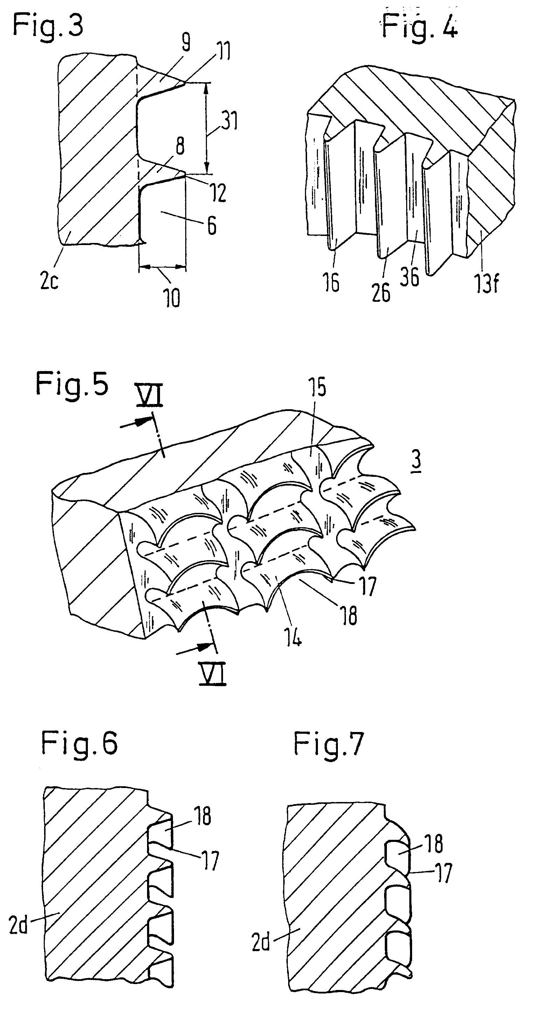

In Figur 3 ist die in einem Bearbeitungsschritt I an einem Schalenrohling 2c erzeugte Geometrie der Einstiche 6 und der Schultern 8 gezeigt. Die Schultern 8 weisen einen spitzwinkligen Dreieckquerschnitt 9 auf und haben zwischen zwei benachbarten Spitzen 11, 12 einen Abstand 31 der etwa dem doppelten Betrag ihrer Höhe 10 entspricht. Um für das Einwachsen und Verankern günstige Verhältnisse zu schaffen, wird die Höhe 10 zwischen 0,3 und 2 mm gewählt. Ein typischer Wert für die Höhe 10 ist 1 mm.

Aus Figur 4 ist die Form der Rippen 16 an einem Tiefziehwerkzeug 13f sichtbar. Die Rippen 16 haben die Form eines Satteldachs 26, dessen First verrundet ist und stehen mit ihrer Mittelebene senkrecht von einer Grundfläche 36 weg, wobei die Mittelebenen parallel zur Einbring- und Befestigungsrichtung 40 verlaufen. Diese Rippen erzeugen durch plastische Verformung die in Figur 5 sichtbaren Gräben 15, welche die Schultern in Schulterabschnitte 14 aufteilen. Da das Tiefziehwerkzeug entgegen der Einbring- und Befestigungsrichtung auf die Verankerungsfläche 3 auffährt, werden die Schulterränder 17 der Schulterabschnitte 14 herabgezogen, sodass entgegen der Einbring- und Befestigungsrichtung 40 Becherformen 18 entstehen. Figuren 6 und 7 zeigen an einem Ausschnitt eines Schalenrohlings 2d unterschiedlich weit herabgezogene Schulterränder 17. Die Geometrie der Becherform 18 ist mit der Veränderung der Schultern 8 und der Rippen 16 steuerbar. Je kleiner im weiteren die Winkel α der Verankerungsfläche 3 zur Einbring- und Befestigungsrichtung 40 sind, desto weiter werden die Schulterränder 17 herabgezogen. Im Fall der äusseren Hüftgelenkschale in Figur 1 bedeutet dies, dass die typischen Becherformen 18 am ausgeprägten dort entstehen, wo sie am meisten gegen das Ausziehen benötigt werden, nämlich zum Äquator 32 hin.

In Figur 8 ist eine Arbeitsfolge für die Formgebung an einer äusseren Hüftgelenkschale gezeigt, ohne eventuell dazwischenliegende Wärmebehandlungen zu berücksichtigen.

In einem Vorschritt V1 wird eine Rondelle 1 aus Titanblech in eine Tiefziehform 13b eingelegt, mit einem Niederhalter 24 fixiert und durch einen Werkzeugstempel 13a zu einer Schale gezogen, die mit einem Ausstosser 22 in Richtung der Polachse 4 ausgeworfen wird. Dieser Schalenrohling wird in einem Vorschritt V2 auf einen Stützkörper 13c aufgelegt und erhält fakultativ mit einem Tiefziehwerkzeug 13d, welches mit Buckeln 25 in Längsrichtung versehen ist, zusätzliche Längsrillen 29 für die spätere Drehsicherung im Knochenbett. In einem Zwischenschritt Z werden Aufnahmeflächen im Bereich des Äquators angedreht. Eine Drehrichtung 27, eine Drehachse 28, ein Spannwerkzeug 23 und ein Drehstahl 7 sind nur symbolisch angedeutet.

In einem Verfahrensschritt I wird ein Schalenrohling 2b an seinen Aufnahmeflächen am Äquator mit einem Spannwerkzeug 23 eingespannt und auf der Verankerungsfläche mit Einstichen 6 versehen. Die Drehachse 28 in der Richtung der Polachse 4, die Drehrichtung 27 und der Drehstahl 7 sind symbolisch angedeutet. Die Geometrie der Einstiche 6 und der Schultern 8 wurde bereits vorher beschrieben. Der Drehstahl 7 wird hier um den Kugelmittelpunkt 5 der Aussenschale geschwenkt. Es können aber auch Formstähle auf verschiedenen Breiten der Kugelschale eingesetzt werden. Ein so vorbereiteter Schalenrohling 2c, der Schultern quer zur Einbring- und Befestigungsrichtung 40 aufweist wird in einem Tiefziehwerkzeug auf einen Stützkörper 13e aufgesetzt und in einem Verfahrensschritt II mit einer Tiefziehform 13f, welche eine Halbkugelform mit in den Meridianen vorstehenden Rippen 26 aufweist, an seinen Schultern 8 verformt. Die entgegen der Einbring- und Befestigungsrichtung auftreffenden Rippen 26 erzeugen die in Figur 5 gezeigten Gräben 15 und die Schulterabschnitte 14 mit der Becherform 18. In einem Nachfolgeschritt N wird der Schalenrohling 2d an seinen Aufnahmeflächen am Äquator mit einem Spannwerkzeug 23 aufgenommen, um die Innenkontur 19 fertig zu drehen. Die Drehachse 28 in Richtung der Polachse 4, der Drehstahl 7 und die Drehrichtung 27 sind nur symbolisch angedeutet.

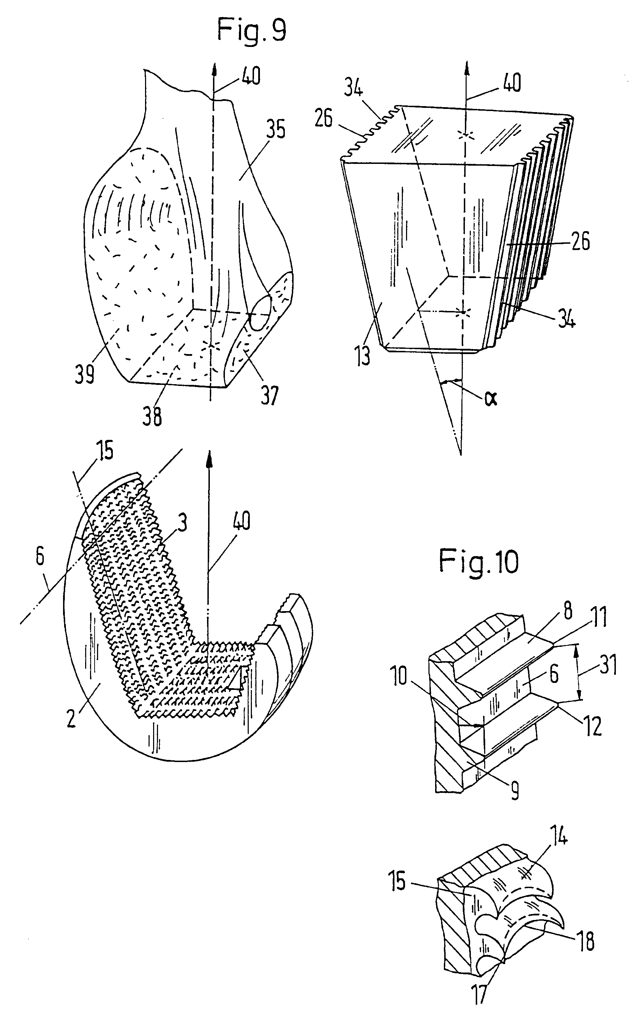

In Figur 9 sind an einem Femurstumpf 35 Flächen 37, 38, 39 durch Resektion erzeugt worden, an denen die Verankerungsflächen 3 eines als Rohling 2 gezeigten Oberteils eines Kniegelenks in einer Einbring- und Befestigungsrichtung 40 eingreifen sollen. Ein zugehöriges Presswerkzeug 13 besitzt Grundflächen 34, die den Resektionsflächen 37, 38, 39 entsprechen und die Rippen 26 aufweisen, welche mit ihrer Mittelebene senkrecht zur Grundfläche 34 und parallel zur Einbring- und Befestigungsrichtung 40 verlaufen. Quer zur Einbring- und Befestigungsrichtung 40 sind am Rohling 2 Einstiche 6 angebracht, die Schultern 8 stehenlassen. Im oberen Teil von Figur 10 sind die Schultern 8 mit einem Einstich 6 vergrössert gezeigt. Die Schulterhöhe 10 beträgt zwischen 0,3 und 2 mm während der Abstand 31 der Schulterspitzen 11, 12 etwa dem zweifachen Betrag der Schulterhöhe 10 entspricht. Der Querschnitt der Schultern 8 entspricht einem spitzwinkligen Dreiecksquerschnitt 9.

Beim Einfahren des Werkzeugs 13 in den Rohling 2 entstehen in den Verankerungsflächen 3 Gräben 15, welche die Schultern 8 wie im unteren Teil von Figur 10 gezeigt in Schulterabschnitte 14 unterteilen und entgegen der Einbring- und Befestigungsrichtung 40 die Schulterränder 17 herabziehen und Becherformen 18 erzeugen. Je kleiner der Winkel α zwischen Grundfläche 34 und der Einbring- und Befestigungsrichtung 40 ist, desto ausgeprägter sind die Becherformen.

## Patentansprüche

1. Verfahren zur Erzeugung von äusseren Verankerungsflächen (3) an Gelenkimplantaten aus einem plastisch verformbaren Metall, wobei die Verankerungsflächen in einem Winkel α zwischen 0 und 90 Grad zu einer Einbring- und Befestigungsrichtung (40) des Gelenkimplantats stehen, dadurch gekennzeichnet, dass in einem Schritt I an einem Rohling (2) in den Verankerungsflächen Einstiche (6) quer zur Einbring- und Befestigungsrichtung durch Spanen, Giessen oder Fressen erzeugt werden, sodass spitzwinklige Schultern (8) stehen bleiben, welche eine Höhe (10) in einem Betrag zwischen 0,3 und 2 mm aufweisen, während der Abstand zweier benachbarter Schulterspitzen (11, 12) annähernd dem Betrag der zweifachen Höhe (10) entspricht, und dass in einem Schritt II der Rohling (2) mit einem Presswerkzeug (13), dessen Grundfläche (34) der Hüllfläche an den Verankerungsflächen (3) des Rohlings (2) entspricht und aus dessen Grundfläche (34) Rippen (26) vorstehen, welche mit ihren Mittelebenen senkrecht zur Grundfläche (34) und parallel zur Einbring- und Befestigungsrichtung (40) verlaufen, durch Pressen in der Einbring- und Befestigungsrichtung (40) soweit verformt wird, dass mit den Rippen (26) quer zu den Schultern (8) Gräben (15) erzeugt werden, welche Schulterabschnitte (14) stehen lassen, wobei die Schulterränder (17) im Bereich der Gräben (15) entgegen der Einbring- und Befestigungsrichtung (40) plastisch deformiert werden, um auf diese Weise aus den Schulterabschnitten (14) Becherformen (18) zu erzeugen, welche entgegen der Einbring- und Befestigungsrichtung (40) geöffnet sind.

2. Verfahren zur Erzeugung von äusseren Verankerungsflächen (3) an metallischen Hüftgelenkschalen, die vorzugsweise aus Reintitan bestehen, dadurch gekennzeichnet,
dass in einem Schritt I an einem Schalenrohling (2b) in Form einer annähernd kugelförmigen Halbschale durch Drehen um deren Polachse (4) Einstiche (6) mit einen Stahl (7) erzeugt werden, die zwischen sich spitzwinklige Schultern stehen lassen, deren Höhe (10) einen Betrag zwischen 0,3 und 2 mm aufweist, während der Abstand (31) zweier Schulterspitzen (11, 12) annähernd dem Betrag der zweifachen Höhe (10) entspricht, und dass in einen Schritt II der Schalenrohling (2c) in einem Tiefziehwerkzeug (13e, 13f) mit halbkugeliger Aussparung, welche in Richtung zur Polachse (4) auf Meridianen vorstehende Rippen (26) aufweist, die so bemessen sind, dass sie beim Pressen des Schalenrohlings in der Richtung der Polachse (4) quer durch die Schultern (8) Gräben (15) mit dazwischenliegenden Schulterabschnitten (14) erzeugen, dass sie mit zunehmender Annäherung von der Polseite an den Äquator (32) der Schale die Schulterränder (17) im Bereich der Gräben (15) zum Äquator hin plastisch deformieren und dass auf diese Weise aus Schulterabschnitten (14) zum Äquator hin geöffnete Becherformen (18) erzeugt werden.

3. Verfahren nach Anspruch 2, dadurch gekennzeichnet, dass die Rippen (26) auf den Meridianen des Tiefziehwerkzeuges (13e, 13f) einen Abstand zwischen 0,5 und 4 mm zueinander aufweisen und die Form eines spitzwinkligen Satteldachs (16) aufweisen.

4. Verfahren nach Anspruch 2 oder 3, dadurch gekennzeichnet, dass in einem Vorschritt V1 der Schalenrohling (2a) aus einer Blechrondelle (1) in einem Tiefziehwerkzeug (13a, 13b, 24) geformt wird.

5. Verfahren nach einem der Ansprüche 2 bis 4, dadurch gekennzeichnet, dass in einem Vorschritt V2 mit einem Tiefziehwerkzeug (13c, 13d, 22) mindestens drei Längsrillen (29) am Umfang des Äquators angebracht werden.

6. Verfahren nach einem der Ansprüche 2 bis 5, dadurch gekennzeichnet, dass in einem Zwischenschritt Z Aufnahmeflächen (20, 21) für Spannmittel (23) der späteren Bearbeitungsschritte angebracht werden.

7. Verfahren nach einem der Ansprüche 2 bis 5, dadurch gekennzeichnet, dass in einem Nachfolgeschritt N eine Innenkontur (19) der metallischen Hüftgelenkschale zur Aufnahme und Verankerung einer Pfanne fertig bearbeitet wird.

## Claims

1. A process for the production of outer anchoring faces (3) on joint implants made from a plastically deformable metal, whereby the anchoring faces are at an angle α of between 0 and 90 degrees to an insertion and attachment direction (40) of the joint implant,
**characterised in that** in a step I on a blank (2) recesses (6) are produced in the anchoring faces at right angles to the insertion and attachment direction by machining, casting or pressing, so that acute-angled shoulders (8) remain, which have a height (10) of between 0.3 and 2 mm, while the distance between two adjacent shoulder points (11, 12) roughly corresponds to twice the height (10),
**and in that** in a step II, with a pressing tool (13), the base (34) of which corresponds to the shell face on the anchoring faces (3) of the blank (2) and from the base (34) of which ribs (26) protrude, which extend with their centre planes perpendicularly to the base (34) and parallel to the insertion and attachment direction (40), the blank (2) is deformed by pressing in the insertion and attachment direction (40) so that troughs (15), which leave shoulder portions (14), are produced with the ribs (26) at right angles to the shoulders (8), the shoulder edges (17) in the region of the troughs (15) being plastically deformed against the insertion and attachment direction (40), in order to produce thus cupular shapes (18), which are opened against the insertion and attachment direction (40), from the shoulder portions (14).

2. A process for producing outer anchoring faces (3) on metallic hip joint shells, which are preferably made from pure titanium,
**characterised in that** in a step I on a shell blank (2b) in the form of an approximately spherical half shell by turning around its pole axis (4) recesses (6) are produced, which leave acute-angled shoulders between them, the height (20) of which is between 0.3 and 2 mm, while the distance (31) between two shoulder points (11, 12) corresponds roughly to twice the height (10),
**and in that** in a step II the shell blank (2c) in a deep-drawing tool (13e, 13f) having a hemispherical recess, which comprises ribs (26) protruding towards the pole axis (4) on meridian lines, which ribs are dimensioned so that when pressing the shell blank towards the pole axis (4) they produce troughs (15) with intervening shoulder portions (14), so that with increasing proximity of the pole side to the equator (32) of the shell they plastically deform the shoulder edges (17) in the region of the troughs (15) towards the equator and so that in this way cupular shapes (18) open towards the equator are produced from shoulder portions (14).

3. A process according to Claim 2,
**characterised in that** the ribs (26) on the meridian lines of the deep-drawing tool (13e, 13f) comprise a mutual spacing of between 0.5 and 4 mm and have the shape of an acute-angled ridge roof (16).

4. A process according to Claim 2 or 3,
**characterised in that** in a preliminary step V1 the shell blank (2a) is shaped from a circular sheet blank (1) in a deep-drawing tool (13a, 13b, 24).

5. A process according to one of Claims 2 to 4,
**characterised in that** in a preliminary step V2 at least three longitudinal grooves (20) are provided on the periphery of the equator with a deep-drawing tool (13c, 13d, 22).

6. A process according to one of Claims 2 to 5,
**characterised in that** in an intermediate step Z mounting faces (20, 21) are provided for clamping means (23) for the subsequent machining steps.

7. A process according to one of Claims 2 to 5,
**characterised in that** in a subsequent step N an inner contour (19) of the metallic hip joint shell is finished to receive and anchor an acetabulum.

## Revendications

1. Procédé pour produire des surfaces d'ancrage extérieures (3) à des implants d'articulation en un métal plastiquement déformable, où les surfaces d'ancrage s'étendent selon un angle α compris entre 0 et 90 degrés à une direction d'insertion et de fixation (40) de l'implant d'articulation, caractérisé en ce que sont produites lors d'une étape I à une ébauche (2) dans les surfaces d'ancrage des entailles (6) transversalement à la direction d'insertion et de fixation par enlèvement des copeaux, coulée ou pression, de façon que subsistent des épaulements (8) à angle en pointe qui ont une hauteur (10) d'une valeur comprise entre 0,3 et 2 mm, tandis que l'écart entre deux pointes d'épaulement avoisinantes (11,12) correspond approximativement à la valeur de la hauteur double (10), et qu'il est déformé lors d'une étape II l'ébauche (2) avec un outil de pression (13) dont la surface de base (34) correspond à la surface d'enveloppe aux surfaces d'ancrage (3) de l'ébauche (2) et de la surface de base (34) de laquelle dépassent des nervures (26) qui s'étendent avec leurs plans médians perpendiculairement à la surface de base (34) et parallèlement à la direction d'insertion et de fixation (40), par pression dans la direction d'insertion et de fixation (40) suffisamment pour que soient produits avec les nervures (26) transversalement aux épaulements (8) des fossés (15) qui laissent subsister des tronçons d'épaulement (14), où les bords d'épaulement (17) sont déformés plastiquement au voisinage des fossés (15) contre la direction d'insertion et de fixation (40) pour produire de cette manière à partir des tronçons d'épaulement (14) des formes en auge (18) qui sont ouvertes contre la direction d'insertion et de fixation (40).

2. Procédé pour produire des surfaces d'ancrage extérieures (3) à des coques acétabulaires métalliques, qui sont constituées de préférence de titane pur, caractérisé en ce que sont produites lors d'une étape I à une ébauche de coque (2b) sous la forme d'une demi-coque approximativement sphérique, par une rotation autour de son axe polaire (4), des entailles (6) avec un outil de tournage (7) qui laissent subsister entre celles-ci des épaulements à angle pointu dont la hauteur (10) a une valeur comprise entre 0,3 et 2 mm, tandis que l'écart (31) entre deux pointes d'épaulement (11,12)correspond approximativement à la valeur de la hauteur double (10), et en ce que lors d'une étape II, l'ébauche de coque (2c), dans un outil d'emboutissage profond (13e, 13f) avec un évidemment demi-sphérique, qui présente en direction de l'axe polaire (4) des nervures (26) faisant saillie sur des méridiens, qui sont dimensionnées de façon qu'elles produisent lors de la compression de l'ébauche de coque en direction de l'axe polaire (4) transversalement à travers les épaulements (8) des fossés (15) avec des tronçons d'épaulement (14) situés entre ceux-ci, que lors d'une approche de plus en plus grande du côté polaire à l'équateur (32) de la coque, elles déforment plastiquement les bords d'épaulement (17) au voisinage des fossés (15) vers l'équateur et que sont produites de cette manière à partir des tronçons d'épaulement (14) des formes en auge (18) ouvertes vers l'équateur.

3. Procédé selon la revendication 2, caractérisé en ce que les nervures (26) sur les méridiens de l'outil d'emboutissage profond (13e, 13f) présente un écart entre elles compris entre 0,5 et 4 mm et ont la forme d'une toiture à deux versants (16) à angle en pointe.

4. Procédé selon la revendication 2 ou 3, caractérisé en ce que lors d'une étape préalable V1, l'ébauche de coque (2a) est formée à partir d'une rondelle en tôle (1) dans un outil d'emboutissage profond (13a, 13b, 24).

5. Procédé selon l'une des revendications 2 à 4, caractérisé en ce que sont ménagés lors d'une étape préalable V2 avec un outil d'emboutissage profond (13c, 13d, 22) au moins trois rainures longitudinales (29) sur le pourtour de l'équateur.

6. Procédé selon l'une des revendications 2 à 5, caractérisé en ce que sont réalisés lors d'une étape intermédiaire Z des surfaces de réception (20,21) pour des moyens de serrage (23) des étapes d'usinage ultérieures.

7. Procédé selon l'une des revendications 2 à 5, caractérisé en ce que lors d'une étape subséquente N, un contour intérieur (19) de la coque acétabulaire métallique est soumis à un usinage final pour la réception et l'ancrage d'une coque.
